# EUROPÄISCHE PATENTANMELDUNG

(11) **EP 4 403 128 A1**
(43) Veröffentlichungstag der Anmeldung: **24.07.2024**
(21) Anmeldenummer: 24152827.2
(22) Anmeldetag: 19.01.2024
(51) Int. Cl.: A61B 34/20, A61B 34/30, A61B 34/00, A61B 90/00, A61B 90/20, A61B 17/00

(54) **INTRAOPERATIVES CHIRURGISCHES MESSSYSTEM UND MESSVERFAHREN**

(30) Priorität: 23.01.2023 DE 102023101578
(71) Anmelder: B. Braun New Ventures GmbH, 79110 Freiburg im Breisgau (DE)
(72) Erfinder: SARVESTANI, Amir, 79104 Freiburg (DE); STAWIASKI, Jean, 79199 Kirchzarten (DE)
(74) Vertreter: Winter, Brandl - Partnerschaft mbB

(57) **Zusammenfassung**

Die Offenbarung betrifft ein chirurgisches Messsystem (1) zur zeitaktuellen intraoperativen räumlichen Messung und Darstellung von geometrischen Streckenlängen, mit: einer chirurgischen Visualisierungseinheit (2), einem Navigationssystem (6), das zumindest eine Instrumentenspitze (12) eines medizinischen Instruments (10) nachverfolgt, einer visuellen Darstellungsvorrichtung (16), und einer Steuereinheit (20), die dafür angepasst ist: über das Navigationssystem (6) zumindest eine Position einer Instrumentenspitze (12) des navigierten Instruments (10) zu erfassen, eine digitale Überlagerungsdarstellung (22) zu erstellen mit der intrakorporalen Aufnahme (4) und einer virtuelle Messvorrichtung (26) mit zumindest einer Abstandsmarkierung (32) und/oder einer Abstandszahl (34), die einem realen Abstand in der intrakorporalen Aufnahme (4) entspricht und dessen Nullpunkt (14) die Instrumentenspitze (12) bildet, wobei die virtuelle Messvorrichtung (26) parallel zu der Aufnahmeebene (5) angeordnet ist oder ein räumlicher Endpunkt (36) definierbar ist, wobei die Verbindung beider Punkte (14, 36) eine Gerade in Form eines virtuellen Lineals (28) bilden, und diese erstellte Überlagerungsdarstellung (22) mit zumindest der virtuellen Messvorrichtung (26) über die Darstellungsvorrichtung (16) auszugeben. Daneben betrifft die Offenbarung ein Verfahren sowie ein computerlesbares Speichermedium und Computerprogramm gemäß den nebengeordneten Ansprüchen.

## Beschreibung

### Technisches Gebiet

Die vorliegende Offenbarung betrifft ein chirurgisches intraoperatives Messsystem zur zeitaktuellen intraoperativen räumlichen Messung und Darstellung von geometrischen Streckenlängen oder Maßen oder Abständen mit: einer chirurgischen Visualisierungseinheit, das dafür angepasst ist eine intrakorporale Aufnahme eines Eingriffsbereichs in einer Aufnahmeebene zu erstellen, einem Navigationssystem, das dafür angepasst ist, über ein Trackingsystem zumindest eine Instrumentenspitze eines medizinischen Instruments oder medizinischen Geräts (im dreidimensionalen Raum relativ zu dem Patienten) nachzuverfolgen, welches für den Eingriff im Eingriffsbereich einsetzbar ist, um mittels der Instrumentenspitze einen Nullpunkt für die Messung zu definieren, und einer visuellen Darstellungsvorrichtung, insbesondere einem OP-Monitor, zur visuellen Ausgabe einer Darstellung mit zumindest der erstellten intrakorporalen Aufnahme. Es kann vorstehend auch davon gesprochen werden, dass die Offenbarung ein chirurgisches Navigationssystem mit einem (integrierte) intraoperativen Messsystem mit entsprechenden Merkmalen betrifft. Daneben betrifft die vorliegende Offenbarung ein Messverfahren und ein computerlesbares Speichermedium sowie ein Computerprogramm gemäß den Oberbegriffen der nebengeordneten Ansprüche.

### Hintergrund der Offenbarung

In der Neurochirurgie ist der Einsatz von Visualisierungseinheiten bzw. Visualisierungssystemen, beispielsweise einem Operationsmikroskop, und von Navigationssystemen für eine präzise Navigation von Instrumenten oder aber auch dem Visualisierungssystem selbst mittlerweile ein immer wichtigerer Bestandteil eines chirurgischen Eingriffs an einem Patienten.

Die Visualisierungssysteme erzeugen dabei eine vergrößerte Ansicht des Patientengewebes, haben jedoch den Nachteil, dass sie keine präzisen speziellen (geometrische) Messungen erlauben. Solche Messungen sind jedoch erforderlich, um eine Größe von anatomischen Strukturen von Interesse wie beispielsweise Blutgefäße, Nerven oder Tumore zu bestimmen und dem Chirurgen bei einem Eingriff zu unterstützen.

Für präzise Messungen von Längen, Abmessungen, Abständen oder ähnlichen Größen können Navigationssysteme verwendet werden. Diese Navigationssysteme nach aktuellem Stand der Technik bieten Messfunktionen "on the fly", also eine schnelle, zeitaktuelle Messung, indem sie ein virtuelles Lineal unmittelbar an der Spitze eines navigierten Instruments anzeigen. Mit diesem virtuellen Lineal können geometrische Messungen entweder entlang der Instrumentenachse (zum Beispiel ein Abstand zu einem Zielgewebe) oder aber genau senkrecht zum Instrument (zum Beispiel Durchmesser eines Ziels) durchgeführt werden. In der Regel werden dabei virtuelle Markierungen angezeigt, die es dem Benutzer ermöglichen, die gewünschten Messungen intuitiv vorzunehmen. Das virtuelle Lineal wird dabei in den Navigationsaufnahmen bzw. -bildern (beispielsweise CT-Aufnahmen oder MRT-Aufnahmen) überlagert und entsprechend über eine Darstellungsvorrichtung ausgegeben. Im Falle eines navigierten Visualisierungssystems (etwa eines navigierten Operationsmikroskops) kann das Lineal auch über das Bild des Visualisierungssystems, wie etwa einem Mikroskopbild, gelegt bzw. überlagert werden. Die Bilder des Visualisierungssystems stehen dabei jedoch immer senkrecht zu einer optischen Achse des Visualisierungssystems, das heißt zu einer chirurgischen Sichtachse. Sowohl bei 2D-Aufnahmen als auch bei 3D-Aufnahmen (Bildern) blickt der Benutzer also stets auf ein ebenes Bild, das senkrecht zu seiner Sichtachse liegt. Im Falle der Verwendung eines navigierten Instruments, wie zum Beispiel eines Pointers/ navigierten Zeigers, steht das Instrument jedoch im Allgemeinen nicht senkrecht zu der Bildebene bzw. parallel zu der optischen Achse bzw. der Blickachse, bildet jedoch das Referenzsystem für gerade die Messung. Ein virtuelles Lineal wird also stets an dem Instrument selbst insbesondere in Verlängerung der Instrumentenachse ausgerichtet. Dies hat den Nachteil, dass so räumliche Messungen mit einer Messfunktion vom Typ Lineal im Allgemeinen nicht parallel zu der Bildebene durchgeführt werden können. Der Nutzer muss dafür versuchen das Instrument möglichst parallel zu der Aufnahmeebene zu halten oder senkrecht darauf mit einem virtuellen Messlineal senkrecht zur Instrumentenachse. Aber selbst in dieser Variante kann keine exakte Messung erfolgen, da stets eine ungenaue Ausrichtung des Instruments als auch einer Position eines Ursprungs gegeben ist. Möchte der Benutzer beispielsweise den Durchmesser eines auf dem Bild des Visualisierungssystems sichtbaren Blutgefäßes messen, kann er diesen Parameter nicht direkt mit einem (navigierten) virtuellen Lineal messen.

Eine Alternative zu einem navigierten virtuellen Lineal und der Messung ("on the fly") wäre eine Messfunktion, die auf der Definition von 3D-Punkten im Raum mit einem navigierten Instrument basiert. Durch Auswahl eines Punktes auf einer Seite des Blutgefäßes und eines Punktes auf der anderen Seite kann beispielsweise der Durchmesser des Blutgefäßes bestimmt und über das Navigationssystem berechnet und ausgegeben werden. Allerdings erfordert eine solche Messung vordefinierte Schritte und Interaktionen des Nutzers, was die Messung viel zeitaufwändiger und deutlich weniger intuitiv macht. Insbesondere muss der Benutzer, insbesondere Chirurg, intraoperativ auf Anhieb die richtigen Punkte setzen, wobei jede Korrektur dieser Punkte eine zeitliche Länge eines Eingriffs, welcher oftmals bereits selbst zeitbeschränkt ist, deutlich erhöht, sodass ein Chirurg oftmals von einer solchen Messung absieht und damit ein Risiko für den Patienten erhöht.

Eine weitere neue Technologie für intraoperative Messungen für eine chirurgische Visualisierung basiert darauf, dass für virtuelle Intra-OP-Messungen bei laparoskopischen und robotergestützten Operationen Messungen in 3D durchgeführt werden. Dieses System bietet jedoch keine zeitaktuelle "on-the-fly"-Messung mit einer Messfunktion mittels Lineal, die an einem einzelnen navigierten Instrument (virtuell) angebunden oder zugeordnet werden können.

### Zusammenfassung der Offenbarung

Es ist daher die Aufgabe der vorliegenden Offenbarung die Nachteile aus dem Stand der Technik zu vermeiden oder zumindest zu mindern und insbesondere ein chirurgisches System, ein Verfahren sowie ein computerlesbares Speichermedium und Computerprogramm zur Verfügung zu stellen, das einen medizinischen Fachpersonal wie einem Chirurgen intraoperativ, also während einer Operation, in einer aktuellen Live-Aufnahme an einer vordefinierbaren Position situations- und bedarfsgerecht eine virtuelle Messvorrichtung/ ein virtuelles Messinstrument bereitstellt, welche reale Maße oder Abstände oder Streckenlängen virtuell in die intrakorporale Aufnahme mit einbindet und zwar unabhängig von einer Ausrichtung eines nachverfolgten Instruments wie etwa einem Pointer. Eine weitere Teilaufgabe kann darin gesehen werden, in verschiedenen Ansichts-Modalitäten ein virtuelles Messinstrument (gleichermaßen) und für einen Nutzer intuitiv mit einzubinden. Auch kann eine Teilaufgabe darin gesehen werden, einem Nutzer eine Option einer Definition einer Anordnung einer virtuellen Abstandsanzeige zur Verfügung zu stellen, um unabhängig von der Ausrichtung des nachverfolgten Instruments im Raum beliebig eine virtuelle Abstandsanzeige mit einzubinden.

Die Aufgaben der vorliegenden Offenbarung werden hinsichtlich eines gattungsgemäßen chirurgischen intraoperativen Messsystem erfindungsgemäß durch die Merkmale des Anspruchs 1 gelöst, hinsichtlich eines chirurgischen Messverfahrens erfindungsgemäß durch die Merkmale des Anspruchs 12 gelöst, hinsichtlich eines computerlesbaren Speichermediums erfindungsgemäß durch die Merkmale des Anspruchs 13 gelöst, und hinsichtlich eines Computerprogramms erfindungsgemäß durch die Merkmale des Anspruchs 14.

Ein Grundgedanke der vorliegenden Offenbarung sieht also vor, eine zeitaktuelle Messung ("on-the-fly") unabhängig von der (An-)Winkelung oder Ausrichtung des verwendeten navigierten Instruments zu ermöglichen, und insbesondere parallel zu der Abbildungsebene bzw. Aufnahmeebene des Visualisierungssystems bzw. der Visualisierungseinheit eine virtuelle Messvorrichtung bereitzustellen.

Mit anderen Worten wird ein Messsystem beschrieben, das die folgenden Komponenten aufweist: ein chirurgisches Visualisierungssystem bzw. Visualisierungseinheit (wie insbesondere einem Operationsmikroskop und/oder einem Exoskop und/oder einem Endoskop) für die Erstellung einer intrakorporalen (insbesondere zeitaktuellen) Aufnahme, einem Navigationssystem, und ein durch das Navigationssystem im dreidimensionalen Raum (3D-Raum) nachverfolgtes/ getracktes Instrument mit Instrumentenspitze, sowie einer Steuereinheit (Verarbeitungseinheit/ Prozessoreinheit) und einer Darstellungsvorrichtung, insbesondere einem Display. Die Steuereinheit ist insbesondere speziell angepasst, auch vorzugsweise zur Kalibrierung des Visualisierungssystems, jedoch insbesondere zur Nachverfolgung (tracken) der Position des navigierten Instruments und zur Anzeige seiner Position in bzw. auf der intrakorporalen Aufnahme der Visualisierungseinheit; sowie einer spezielle Anpassung der Steuereinheit zur Darstellung einer virtuellen Messvorrichtung, insbesondere eines virtuellen Lineals in bzw. auf der intrakorporalen Aufnahme des Visualisierungssystems und zwar parallel zu der Aufnahmeebene oder bei zwei definierten Punkten auf einer Geraden zwischen diesen beiden Punkten (wobei ein Punkt den Nullpunkt bildet).

Ein wesentliches Ziel der vorliegenden Offenbarung ist also, dem Benutzer ein (geometrisches) räumliches Messsystem zur Verfügung zu stellen, mit dem er geometrische Abstände von Gewebe auf einem Live-Bild bzw. einer Live-Aufnahme (intrakorporalen Aufnahme) des Patienten schnell, zuverlässig und intuitiv messen kann. Ein navigiertes Instrument wird dabei verwendet, um einen Punkt von Interesse in dem chirurgischen Eingriffsbereich auszuwählen. Ein Navigationssystem lokalisiert dabei die 3D-Position der Spitze des Instruments (im Raum, also ermittelt die X,Y,Z Koordinaten) und nutzt diese als Nullpunkt einer virtuellen Messvorrichtung, insbesondere eines virtuellen Lineals. Ein Kernaspekt der vorliegenden Offenbarung liegt also darin, dass die Messungen auch in einer Ebene parallel zu der Aufnahmeebene des Visualisierungssystems durchgeführt werden können, und damit folglich unabhängig von der Ausrichtung des Instruments relativ zu der Aufnahmeebene bzw. Abbildungsebene sind. Zu diesem Zweck wird die Instrumentenspitze zunächst im 3D-Raum nachverfolgt (X,Y,Z-Position, insbesondere kontinuierlich oder bei Nutzereingabe etwa diskret) und die Instrumentenspitze in der intrakorporalen Aufnahme (also im Bild) des Visualisierungssystems angezeigt. Dann wird insbesondere eine Ebene parallel zu der des Bildgebungssystems erstellt und mit der Spitze des Instruments verbunden. In dieser Ebene können verschiedene Arten von virtuellen Linealformen erstellt und der intrakorporalen Aufnahme (dem Bild) des Visualisierungssystems überlagert werden. Das virtuelle Lineal kann insbesondere linienförmig sein mit Markierungen in bestimmten Abständen (ähnlich eines üblichen Schullineals). Alternativ oder zusätzlich kann das virtuelle Messinstrument (ähnlich Abstandsbereichen eines Punkts in einer topografischen Karte - kreisförmiges Lineal) ringförmig mit Kreisen gestaltet sein, welche die Spitze des Instruments als Ursprung bzw. Nullpunkt des Kreises umgeben, wobei jeder Kreis einen bestimmten Abstand zu der Instrumentenspitze definiert und insbesondere jeweils gleich voneinander beabstandet sind, beispielsweise alle 5mm im Durchmesser. Im Falle des linienförmigen Lineals kann der Benutzer das insbesondere Instrument drehen, und die Steuereinheit kann dafür angepasst sein, die Ausrichtung des Instruments zu erfassen und hierüber auch das virtuelle Lineal mitzudrehen, um eine Messung in einem bestimmten Bereich vorzunehmen. Im Falle des kreisförmigen Lineals ist keine Drehung des Instruments erforderlich. In beiden Fällen ist keine weitere Interaktion des Benutzers erforderlich, um eine Messung durchzuführen, sondern lediglich das Ablesen der Markierungen an dem betreffenden Punkt.

Auch kann der Benutzer gemäß der einen Alternative zwei Punkte mit einem navigierten Instrument definieren, wobei beide 3D-Punkte (im Raum) sind, die in der Aufnahme/ im Bild des Visualisierungssystems erfasst werden. Nachdem der zweite Punkt definiert wurde, zeigt das Messsystem ein Lineal mit der Länge zwischen den beiden Punkten und/oder eine Skala mit Strichen an. In diesem Fall ist die Messung im Allgemeinen nicht auf die Bildebene beschränkt, da die beiden Punkte in zwei verschiedenen Ebenen liegen können. Eine Abweichung hiervon wäre beispielsweise ähnlich einer gummibandartigen Funktion, bei der nach der Festlegung des ersten Punktes eine Art Gummiband mit einer Skala zwischen dem festen ersten Punkt und der aktuellen Spitze des Instruments erscheint. Dies würde es ermöglichen, Messungen vorzunehmen, ohne einen zweiten Punkt zu definieren, wobei nach erstmaliger Definition eines Startpunkts die Instrumentenspitze als dynamischer zweiter Punkt agiert.

Das Messsystem erfordert vorzugsweise ein kalibriertes Visualisierungssystem bzw. Visualisierungseinheit, das heißt die Bildparameter sind mit einer externen Referenz bekannt, die zur Nachverfolgung des Instruments verwendet wird. Die externe Referenz kann beispielsweise das Gehäuse des Visualisierungssystems sein, beispielsweise das Gehäuse des Mikroskopkopfes. Optional kann das Gehäuse des Visualisierungssystems durch ein Navigationssystem nachgeführt werden. Alternativ oder zusätzlich kann auch die intrakorporale Aufnahme des kalibrierten Visualisierungssystems verwendet werden, um die Position des Instruments mit Hilfe von Machine-Vision (maschinellem Sehen) zu verfolgen.

Insbesondere kann das chirurgische Messsystem aufweisen: eine Visualisierungsvorrichtung und ein Navigationssystem, wobei die Aufnahme des Visualisierungssystems vorzugsweise kalibriert ist, ein navigiertes bzw. nachverfolgtes Instrument, um einen Startpunkt bzw. Nullpunkt einer räumlichen Messung zu definieren, wobei der Aufnahme/ dem Bild des Visualisierungssystems eine virtuelle Messvorrichtung (insbesondere ein virtuelles Mess-Lineal) überlagert ist, wobei die Orientierung, insbesondere die Lage, (Struktur) des virtuellen Messvorrichtung wie etwa Lineals parallel zu der Aufnahmeebene, insbesondere in der Aufnahmeebene, des Visualisierungssystems liegt, sodass die Aufnahmeebene des Visualisierungssystems unabhängig von der Ausrichtung des Instruments zu der Aufnahmeebene der Visualisierungsvorrichtung ist und der Benutzer anhand der Form/ Struktur des virtuellen Messvorrichtung (etwa Lineals) einen Endpunkt der räumlichen Messung bestimmen kann.

Mit ganz anderen Worten wird ein vorliegend ein chirurgisches intraoperatives Messsystem zur zeitaktuellen intraoperativen räumlichen Messung und Darstellung von geometrischen Streckenlängen oder Maßen oder Abständen zur Verfügung gestellt mit: einer chirurgischen Visualisierungseinheit, das dafür angepasst ist eine intrakorporale Aufnahme eines Eingriffsbereichs in einer Aufnahmeebene zu erstellen, einem Navigationssystem, das dafür angepasst ist, über ein Trackingsystem zumindest eine Instrumentenspitze eines medizinischen Instruments (im dreidimensionalen (3D) Raum relativ zu dem Patienten) nachzuverfolgen, welches für den Eingriff im Eingriffsbereich einsetzbar ist, um mittels der Instrumentenspitze einen Nullpunkt für die Messung zu definieren, und einer visuellen Darstellungsvorrichtung, insbesondere einem OP-Monitor, zur visuellen Ausgabe einer Darstellung mit zumindest der erstellten intrakorporalen Aufnahme. Das Messsystem weist ferner eine Steuereinheit auf, die dafür angepasst ist: - vorzugsweise die Visualisierungseinheit zu kalibrieren, - über das Navigationssystem zumindest eine Position einer Instrumentenspitze des navigierten Instruments zu erfassen, insbesondere eine Lage des Instruments mit Instrumentenspitze zu erfassen, - eine digitale Überlagerungsdarstellung zu erstellen mit zumindest der intrakorporalen Aufnahme sowie insbesondere zumindest einer virtuellen Referenzdarstellung der Instrumentenspitze an der nachverfolgten Position, insbesondere einem Punkt oder Kreis als Referenzdarstellung der Instrumentenspitze oder einer Linie mit Ursprung oder Nullpunkt an der Instrumentenspitze entlang einer Längsachse des Instruments bei Erfassung einer Lage des Instruments, - in der erstellten Überlagerungsdarstellung ferner eine virtuelle Messvorrichtung, insbesondere ein virtuelles Lineal, mit zumindest einer Abstandsmarkierung und/oder einer Abstandszahl einzufügen, die einem realen Abstand in der intrakorporalen Aufnahme entspricht und dessen Nullpunkt die Instrumentenspitze bildet/ ist, wobei die virtuelle Messvorrichtung: parallel zu der Aufnahmeebene angeordnet ist, insbesondere in der Aufnahmeebene selbst liegt, um eine Abstandsmessung in der Aufnahmeebene anzuzeigen, (sodass die virtuelle Messvorrichtung unabhängig von der Ausrichtung des Instruments zu der Aufnahmeebene ist,) oder mittels dem nachverfolgten Instrument neben dem Nullpunkt noch ein räumlicher Endpunkt definierbar ist, welche beide die Gerade für die virtuelle Messvorrichtung in Form eines Lineals bilden, und - diese erstellte Überlagerungsdarstellung mit zumindest der virtuellen Messvorrichtung über die Darstellungsvorrichtung auszugeben.

Vorliegend meint der Begriff "Aufnahmeebene" eine Fläche, welche orthogonal auf eine optische Achse der Visualisierungseinheit steht. Bei etwa einem 2D (zweidimensionalen) Endoskop als Visualisierungseinheit mit einem endständigen optischen System wird ein zweidimensionales Bild oder Video aufgenommen wobei das Bild bzw. Video senkrecht auf die optische Achse steht und damit die Aufnahmeebene definiert.

Der Begriff "virtuelle Messvorrichtung" oder "virtuelles Messinstrument" meint vorliegend eine digitale visuelle Darstellung und Einbindung einer solchen geometrischen virtuellen Struktur, die ein Ablesen eines räumlichen Abstands bzw. mehrerer Abstände (direkt in der intrakorporalen Aufnahme) ermöglicht und gewissermaßen skaliert in der vordefinierten Ausrichtung mit eingeblendet wird. Beispielsweise kann ein virtuelles Lineal eingebunden und so skaliert werden, dass es die für die Aufnahme korrekten und realen Abmessungen anzeigt. Wird etwa optisch oder digital mit einem Operationsmikroskop in das Gewebe hineingezoomt, so ändert sich auch entsprechend die Skalierung der virtuellen Messvorrichtung, so dass stets die tatsächlichen Abstände auch angezeigt werden, ähnlich eines CAD-Programms oder einer Weltkarte mit Streckenbalken.

Das Navigationssystem kann insbesondere ein chirurgisches Computer-Tomographie-basiertes Navigationssystem oder ein Magnetresonanztomographiebasiertes (MRT-basiertes) Navigationssystem sein, welches präoperative 3D-Aufnahmen in Form von CT-Aufnahmen oder MRT-Aufnahmen verwendet. Insbesondere ist das Navigationssystem ein Optik-basiertes Navigationssystem, welches mittels einer Navigationskamera optisch Instrumente und optische Marker räumlich nachverfolgt. Alternativ oder zusätzlich kann das Navigationssystem elektromagnetisch (EM) basiert sein und Sensoren für eine Detektion eines EM-Feldes aufweisen. Vorzugsweise kann das Navigationssystem einen präoperativ erstellten und/oder intraoperativ anpassbaren digitalen Operationsplan aufweisen, welcher Annotationen zu 3D-Aufnahmen bereitstellt, etwa eine Trajektorie, etwa mit einem Pfeil gekennzeichnet, und/oder eine Gewebebezeichnung, vorzugsweise mit Gewebebewertung, und/oder eine intraoperative Aufnahme. Das Navigationssystem ist dafür angepasst eine Registrierung des Patienten durchzuführen.

Vorteilhafte Ausführungsformen sind in den Unteransprüchen beansprucht und werden insbesondere nachfolgend erläutert.

Gemäß einer Ausführungsform kann das nachverfolgte Instrument entweder im Bereich seiner Instrumentenspitze ein vordefiniertes optisches Muster und/oder eine vordefinierte geometrische Form aufweisen, die/das in einer Speichereinheit hinterlegt ist, und die Steuereinheit dafür angepasst sein auf Basis des erfassten optischen Musters oder der geometrischen Form über die intrakorporale Aufnahme der chirurgischen Visualisierungseinheit und mittels Machine Vision (Maschinellem Sehen) die Position der Instrumentenspitze, insbesondere des Lage des Instruments, nachzuverfolgen. Bei einer klassischen Navigation kann vorzugsweise ein starrer Körper an dem Instrument befestigt sein. Bei der Bildverarbeitungsnavigation wird die Position des Instruments entweder mit einem bekannten optischen Muster an der Spitze des Instruments oder ohne Muster anhand der bekannten Form des Instruments lokalisiert. In beiden Fällen muss jedoch die Instrumentenspitze im Bild des Visualisierungssystems bzw. der Visualisierungseinheit sichtbar sein. Mit anderen Worten kann das Navigationssystem dafür angepasst sein, mittels maschinellem Sehen (Machine Vision) zu arbeiten, wobei das Navigationssystem ferner dafür angepasst ist, die (zeitaktuelle Live-)Aufnahme des Visualisierungssystems zu verwenden, um ein Instrument nachzuverfolgen, wobei das nachverfolgte Instrument insbesondere entweder mit einem (bekannten) vordefinierten optischen Muster und/oder mit einer (bekannten) vordefinierten Geometrie ausgestattet ist.

Gemäß einer weiteren Ausführungsform kann das Messsystem als nachverfolgtes Instrument einen Zeiger/Pointer und/oder einen Saugschlauch und/oder ein chirurgisches Instrument aufweisen. Mit diesen kann eine Position im Raum für zumindest den Nullpunkt definiert werden. Das nachverfolgte Instrument kann also insbesondere ein chirurgischen (Navigations-)Pointer/ Zeiger oder ein Saugrohr oder ein chirurgisches Instrument sein.

Insbesondere kann das Messsystem als Visualisierungssystem ein Operationsmikroskop und/oder ein chirurgisches Exoskop und/oder ein chirurgisches Endoskop aufweisen. Damit kann das chirurgische Messsystem, das ein 2D- oder 3D-Visualisierungssystem wie ein Endoskop und/oder ein Exoskop und/oder ein Mikroskop neben einem Navigationssystem und einem nachverfolgten Instrument aufweist, dafür verwendet werden unabhängig von der Winkelstellung des Instruments räumliche Messungen wie geometrische Abstände parallel zu der Bildebene des Visualisierungssystems durchzuführen.

Gemäß einer weiteren Ausführungsform kann das Messsystem ferner einen chirurgischen Roboter aufweisen, an dessen Roboterarm die Visualisierungseinheit navigiert angebunden ist. Das Visualisierungssystem ist also vorzugsweise an einem Roboterarm montiert. Hierdurch kann die Visualisierungseinheit insbesondere aktiv bewegt und ausgerichtet werden.

Vorzugsweise kann das gesamte Messsystem in nur einem einzigen fahrbaren Wagen integriert sein. Mit anderen Worten kann das Messsystem als auf einem einzigen Wagen fahrbaren Modul integriert sein, so dass mit nur einem Wage die gesamte Funktionalität gewährleistet ist.

Vorzugsweise können das Visualisierungssystem und das Navigationssystem in einem einzigen medizinischen (bewegbaren) Wagen integriert sein oder jeweils ein Teil von zwei oder mehr separaten Wagen sein. Beispielsweise kann das Visualisierungssystem an bzw. auf einem ersten Wagen integriert sein und das Navigationssystem auf einem separaten, zweiten Wagen.

Gemäß einer weiteren Ausführungsform kann in der Überlagerungsdarstellung eine Nebeneinanderdarstellung von der intrakorporalen Aufnahme und zudem noch einer Navigationsansicht erstellt sein, wobei die virtuelle Messvorrichtung in der intrakorporalen Aufnahme zudem auch noch lagekorrekt in der Navigationsansicht eingeblendet wird, um für einen Nutzer zwei Ansichtsmodalitäten mit jedoch der gleichen virtuellen Messvorrichtung bereitzustellen. Insbesondere kann das virtuelle Lineal sowohl auf bzw. in den Navigationsbildern als auch auf bzw. in den Bildern des Visualisierungssystems angezeigt werden.

Vorzugsweise kann das Navigationssystem eine externe Navigationskamera aufweisen, welche das Instrument mit Instrumentenspitze nachverfolgt.

Gemäß einer bevorzugten Ausführungsform kann das Messsystem ferner eine Eingabeeinheit aufweisen, insbesondere die Darstellungsvorrichtung in Form eines Touchdisplays ausgebildet sein, und die Steuereinheit dafür angepasst sein über eine Nutzereingabe der Eingabeeinheit: eine Abstandsbemaßung des virtuellen Messinstruments einzustellen, insbesondere eine 1 mm oder 5mm Abstandsbemaßung einzustellen, und/oder eine geometrische Struktur der virtuellen Messvorrichtung einzustellen, insbesondere in Form einer Lineallinie mit Abstandsbemaßungen oder in Form von konzentrischen Kreisen, deren Radius sich in gleichen Abständen erhöht, und in der Aufnahmeebene liegt. Insbesondere kann das virtuelle Lineal durch den Benutzer in Form von geometrischen Inkrementen oder Markierungen konfigurierbar sein. Vorzugsweise kann das virtuelle Lineal vom Benutzer, insbesondere über eine Eingabeeinheit wie etwa ein Touchdisplay, in Form von verschiedenen geometrischen Strukturen wie Linien oder Ringen konfiguriert werden, die sich alle in der Bildebene des Visualisierungssystems befinden.

Insbesondere kann die Steuereinheit einen numerischen Abstand zwischen dem Nullpunkt und dem Endpunkt berechnen und diesen numerischen Abstand als Abstandszahl mit einfügen.

Gemäß einer weiteren Ausführungsform kann die Steuereinheit ferner dafür angepasst sein, in der Überlagerungsdarstellung neben der virtuellen Messvorrichtung noch ein zweites virtuelles Lineal in Verlängerung einer Instrumentenlängsachse einzufügen. Auf diese Weise sind Abstandsmessungen bzw. Streckenanzeigen in zwei unterschiedliche Richtungen möglich.

Das Navigationssystem kann insbesondere ein optisches Infrarot-Navigationssystem und/oder ein Navigationssystem mittels Bildverarbeitung und/oder ein elektromagnetisches Navigationssystem sein. Das Navigationssystem kann vorzugsweise eine externe infrarotbasierte Kamera aufweisen.

Optional können präoperative 3D-Bilder (insbesondere dreidimensionale CT-Aufnahmen oder MRT-Aufnahmen) durch die Steuereinheit importiert und mit dem Patienten für eine Navigation registriert werden. Ferner kann insbesondere ein Patiententracker /Patienten-Marker an dem Patienten befestigt sein, der einen starren Bezug (statische Transformation) zu dem Patienten herstellt.

Hinsichtlich eines computerimplementierten chirurgischen intraoperativen Messverfahrens zur zeitaktuellen intraoperativen räumlichen Messung und Darstellung von geometrischen Streckenlängen oder Maßen oder Abständen wird die Aufgabe dadurch gelöst dass dieses die Schritte aufweist: Vorzugsweise kalibrieren einer Visualisierungseinheit, die dafür angepasst ist eine intrakorporale Aufnahme eines Eingriffsbereichs in einer Aufnahmeebene zu erstellen; Erfassen durch ein Navigationssystem, das dafür angepasst ist, über ein Trackingsystem zumindest eine Instrumentenspitze eines medizinischen Instruments (im dreidimensionalen Raum relativ zu dem Patienten) nachzuverfolgen, von zumindest eine Position einer Instrumentenspitze des navigierten Instruments, insbesondere eine Lage des Instruments mit Instrumentenspitze; Erstellen durch eine Steuereinheit einer digitalen Überlagerungsdarstellung mit zumindest der intrakorporalen Aufnahme sowie insbesondere zumindest einer virtuellen Referenzdarstellung der Instrumentenspitze an der nachverfolgten Position, insbesondere einem Punkt oder Kreis als Referenzdarstellung der Instrumentenspitze, mit ferner einer virtuelle Messvorrichtung, insbesondere einem virtuellen Lineal, mit zumindest einer Abstandsmarkierung und/oder einer Abstandszahl, die einem realen Abstand in der intrakorporalen Aufnahme entspricht und dessen Nullpunkt die Instrumentenspitze bildet/ ist, wobei die virtuelle Messvorrichtung parallel zu der Aufnahmeebene angeordnet ist, insbesondere in der Aufnahmeebene selbst liegt, um eine Abstandsmessung in der Aufnahmeebene anzuzeigen, oder mittels dem nachverfolgten Instrument neben dem Nullpunkt noch ein räumlicher Endpunkt definiert wird , welche beide die Gerade für die virtuelle Messvorrichtung in Form eines Lineals bilden, und Ausgeben dieser erstellten Überlagerungsdarstellung mit zumindest der virtuellen Messvorrichtung über eine Darstellungsvorrichtung.

Hinsichtlich eines Computerlesbaren Speichermediums sowie auch hinsichtlich eines Computerprogramms werden die Aufgaben dadurch gelöst, dass dieses Befehle umfasst, die bei einer Ausführung durch einen Computer diesen veranlassen die Schritte des Messverfahrens gemäß der vorliegenden Offenbarung auszuführen.

### Kurzbeschreibung der Figuren

Die vorliegende Offenbarung wird nachfolgend anhand bevorzugter Ausführungsformen unter Bezugnahme auf die begleitenden Figuren näher erläutert.
Fig. 1 eine perspektivische Ansicht eines chirurgischen intraoperativen Messsystems gemäß einer bevorzugten Ausführungsform der vorliegenden Offenbarung,
Fig. 2 eine Ansicht einer Überlagerungsdarstellung des chirurgischen Messsystems aus Fig. 1 mit Nebeneinanderdarstellung von sowohl einer Navigationsansicht als auch einer intrakorporalen Aufnahme mit jeweils eingeblendetem virtuellem Lineal parallel zu der Aufnahmeebene,
Fig. 3 eine schematische perspektivische Ansicht eines virtuellen Lineals mit einmal einer koaxialen Anordnung des Lineals als auch einer Anordnung in der Aufnahmeebene für ein Verständnis der Funktion,
Fig. 4 eine weitere einstellbare Überlagerungsdarstellung des chirurgischen Messsystems aus Fig. 1 mit Nebeneinanderdarstellung von sowohl einer schematischen Navigationsansicht als auch einer intrakorporalen Aufnahme mit jeweils eingeblendetem virtuellem Messinstrument in Form von konzentrischen Kreisen parallel zu der Aufnahmeebene,
Fig. 5 eine Überlagerungsdarstellung des chirurgischen Messsystems aus Fig. 1 mit Nebeneinanderdarstellung von sowohl einer schematischen Navigationsansicht als auch einer intrakorporalen Aufnahme mit jeweils eingeblendetem virtuellem Messinstrument in Form eines virtuellen Lineals zwischen einem ersten definierten Nullpunkt in einer ersten Ebene und einem zweiten Definierten End- oder Stützpunkt in einer zweiten parallelen Ebene, und
Fig. 6 ein schematisches Flussdiagramm eines Messverfahrens gemäß einer bevorzugten Ausführungsform der vorliegenden Offenbarung.

Die Figuren sind schematischer Natur und sollen nur dem Verständnis der Erfindung dienen. Gleiche Elemente sind mit denselben Bezugszeichen versehen. Die Merkmale der verschiedenen Ausführungsformen können untereinander ausgetauscht werden.

### Detaillierte Beschreibung bevorzugter Ausführungsformen

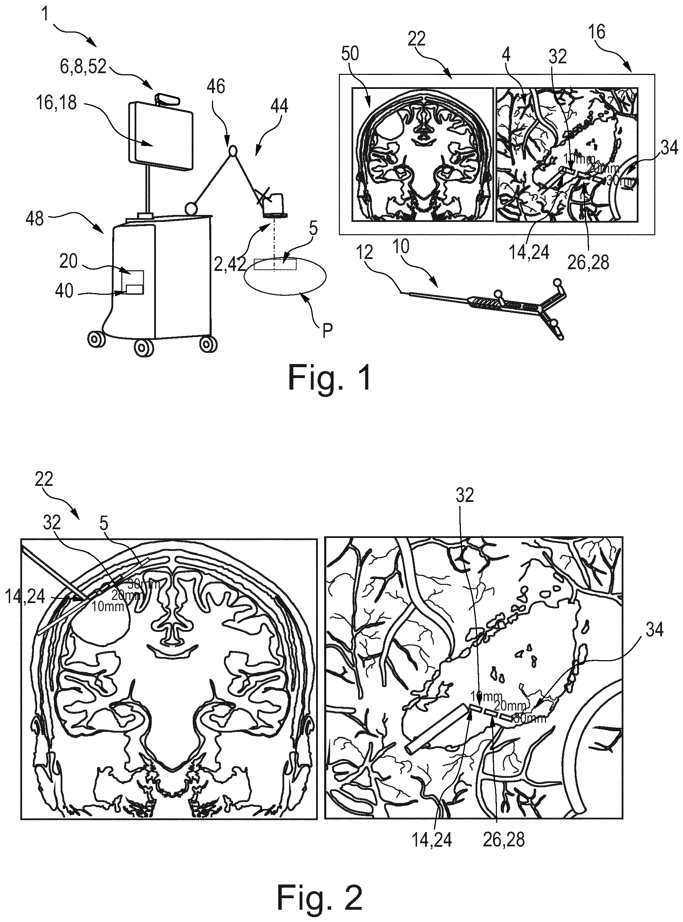

Fig. 1 zeigt in einer perspektivischen Ansicht ein chirurgisches intraoperatives Messsystem 1 (nachstehend nur Messsystem genannt) zur zeitaktuellen intraoperativen räumlichen Messung und Darstellung von geometrischen Streckenlängen oder Maßen oder Abständen. Das Messsystem 1 weist eine chirurgische Visualisierungseinheit 2 auf, die dafür angepasst ist eine intrakorporale Aufnahme 4 eines Eingriffsbereichs eines Patienten P in einer Aufnahmeebene 5 senkrecht zu einer optischen Achse zu erstellen. Ferner weist das Messinstrument ein Navigationssystem 6 auf, das dafür angepasst ist, über ein Trackingsystem 8 zumindest eine Instrumentenspitze 12 eines medizinischen Instruments 10 nachzuverfolgen, welches für den Eingriff im Eingriffsbereich eingesetzt wird, um mittels der Instrumentenspitze 12 einen Nullpunkt 14 für die Messung zu definieren. Auch hat das Messsystem eine visuelle Darstellungsvorrichtung 16, insbesondere einen OP-Monitor 18, zur visuellen Ausgabe einer Darstellung mit zumindest der erstellten intrakorporalen Aufnahme 4.

Im Unterschied zum Stand der Technik weist das Messsystem 1 der vorliegenden Offenbarung eine speziell angepasste Steuereinheit 20 auf. Diese ist konfiguriert die Visualisierungseinheit 2 zu kalibrieren, insbesondere einen hinterlegten Kalibrierungsalgorithmus zu durchlaufen, und dann über das Navigationssystem 6 eine Lage des Instruments 10 mit Position der Instrumentenspitze 12 zu erfassen. Die Steuereinheit 22 ist ferner speziell konfiguriert eine digitale Überlagerungsdarstellung 22 zu erstellen mit sowohl der intrakorporalen Aufnahme 4 als auch einer virtuellen Referenzdarstellung 24 der Instrumentenspitze 12 an der nachverfolgten Position, wobei vorliegend ein Kreis als Referenzdarstellung der Instrumentenspitze gewählt ist. Ferner ist die Steuereinheit dafür angepasst in der erstellten Überlagerungsdarstellung 22 noch eine virtuelle Messvorrichtung 26, insbesondere in Form eines virtuellen Lineals 28 oder von konzentrischen (Abstands-)Kreisen 30 um die Instrumentenspitze herum, mit Abstandsmarkierungen 32 und einer Abstandszahl(angaben) 34 einzufügen, welche dem tatsächlichen, realen Abstand in der intrakorporalen Aufnahme 4 entsprechen. Die Steuereinheit kann ja durch die Navigation bzw. das Navigationssystem 6 stets die geometrischen Beziehungen berechnen und mit einbinden. Der Nullpunkt 14 des virtuellen Messvorrichtung 26 bildet dabei die Instrumentenspitze 12. Die virtuelle Messvorrichtung 26 wird von der Steuereinheit 22 jedoch ganz speziell angeordnet und in der Überlagerungsdarstellung 22 eingefügt, nämlich vorliegend entweder in einem ersten Modus parallel zu der Aufnahmeebene 5, insbesondere in der Aufnahmeebene 5 selbst liegend, um eine Abstandsmessung in der Aufnahmeebene 5 anzuzeigen, oder dass mittels dem nachverfolgten Instrument 10 neben dem Nullpunkt 14 noch ein räumlicher Endpunkt 36 definierbar ist, wobei diese definierten Punkte 14, 36 beide die Gerade für die virtuelle Messvorrichtung 26 in Form eines virtuellen Lineals 28 bilden. Schließlich ist die Steuereinheit dafür angepasst, die erstellte Überlagerungsdarstellung 22 mit zumindest der virtuellen Position der Instrumentenspitze 12 und der virtuellen Messvorrichtung 26 über die Darstellungsvorrichtung 16 auszugeben. Der Patient P ist nur schematisch angedeutet.

Mit dem vorliegend beschriebenen und bereitgestellten Messsystem 1 kann also der Chirurg nicht nur ein virtuelles Lineal in Verlängerung eines Instruments 10 anzeigen lassen, sondern ein virtuelles Lineal in der Aufnahmeebene 5 oder zwischen zwei Punkten setzen und dort die entsprechend skalierte Abstandsbemaßung bzw. Streckenbemaßung (welche der realen Bemaßung entspricht) ausgeben lassen. Das Navigationssystem 6 dient dabei als Basis, um die tatsächlichen Abstände zu bemessen, ähnlich eines CAD-Objekts.

Fig. 2 zeigt schematisch eine Überlagerungsdarstellung 22 des Messsystems 1 aus Fig. 1 mit einem virtuellen Lineal 28 als virtuelle Messvorrichtung 26, das parallel zu der Aufnahmeebene 5 des Visualisierungssystems 2 ausgerichtet ist, wobei der Referenzpunkt bzw. Nullpunkt 14 die 3D-Position (also X,Y,Z Koordinaten) der Instrumentenspitze 12 des navigierten Instruments 10 ist. In der Überlagerungsdarstellung aus Fig. 2 ist auf der linken Seite eine Navigationsansicht 50 mit dem virtuellen Instrument 10 und dem virtuellen Lineal 28 eingeblendet und im rechten Teil der Nebeneinanderdarstellung die zeitaktuelle intrakorporale Live-Aufnahme 4 mit ebenfalls eingeblendetem Instrument 10 und dem virtuellen Lineal 28. Das virtuelle Lineal 28 ist dabei im Bezug zu dem Patienten P gleich ausgerichtet, sodass das (gleiche) virtuelle Lineal 28 einmal lagekorrekt in der Navigationsansicht 50 als auch korrelierend lagekorrekt in der intrakorporalen Aufnahme angezeigt wird, wobei zudem in der Navigationsansicht 50 noch die Aufnahmeebene 5 mit eingeblendet ist, um dem Chirurgen direkt visuell darzustellen, in welcher Ebene er sich relativ zu dem Patienten befindet.

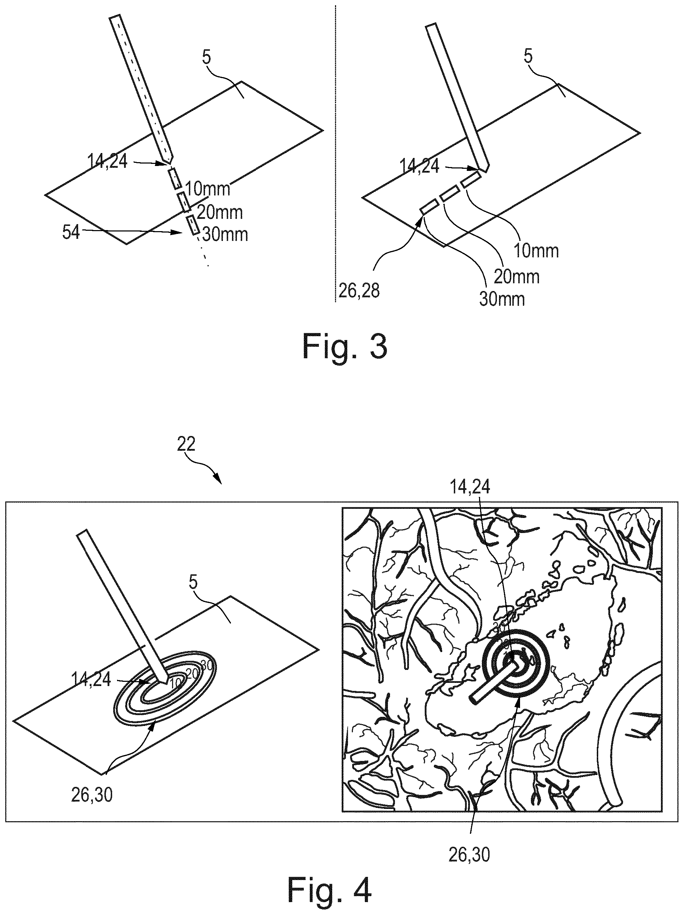

Fig. 3 zeigt eine schematische Darstellung des virtuellen Lineals 28 mit einem navigierten Instrument 10 und einer Aufnahmeebene 5 des Visualisierungssystems 2. Dabei ist in der linken Teilhälfte ein virtuelles Lineal zweites virtuelles Lineal 54 entlang einer Längsachse des Instruments 10 ausgerichtet und in der rechten Teilhälfte der Fig. 3 das virtuelle Lineal 28 parallel zu der Aufnahmeebene 28 ausgerichtet. Während die rechte Teilhälfte ein Aspekt der vorliegenden Offenbarung betrifft, kann die Steuereinheit 20 ferner dafür angepasst sein, zudem auch noch ein zweites virtuelles Lineal 54 mit einzublenden, um dem Chirurgen bei Bedarf sogar noch weitere Messmöglichkeiten anzubieten.

Fig. 4 zeigt anstelle eines linienbasierten virtuellen Lineals 28 aus Fig. 2 eine alternative Überlagerungsdarstellung 22 mit einer solchen Struktur des virtuellen Lineals unter Verwendung von Ringen 30 bzw. Kreisen mit konstantem Abstand zwischen den Ringen selbst, die konzentrisch zur Instrumentenspitze 12 sind Die Kreise bzw. Ringe 30 liegen parallel zu der Aufnahmeebene 5 des Visualisierungssystems 2, und zwar unabhängig von der Ausrichtung/ Orientierung des Instruments 10, so dass der Arzt ohne Drehung des Instruments sehr einfach in alle Richtungen ein Maß ablesen kann. Es werden also an der Instrumentenspitze 12 Abstandsringe parallel zu der Aufnahmeebene 5 angezeigt

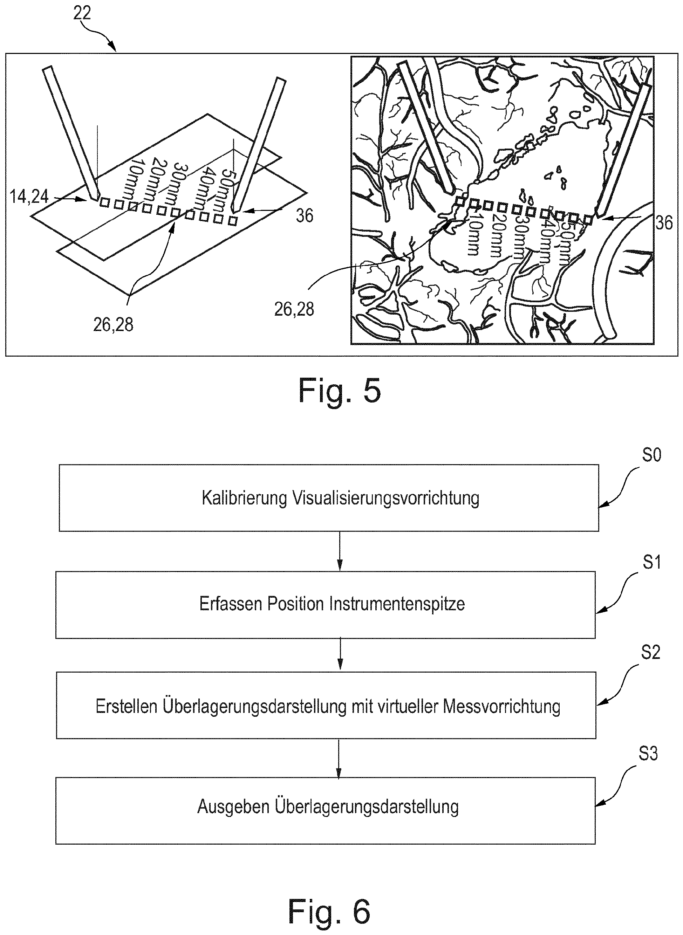

Fig. 5 zeigt eine einstellbare bzw. für den Nutzer auswählbare Funktionsalternative zu den Messungen in der Aufnahmeebene 5, in welcher das virtuelle Lineal 28 auch basierend auf zwei definierten Punkten, einem Nullpunkt 14 und einem Endpunkt 36, in zwei verschiedenen Ebenen erstellt werden kann. Auch diese Alternative der Überlagerungsdarstellung 22 kann das Messsystem 1 bereitstellen. Der Nutzer definiert also zumindest einen ersten Nullpunkt 14, insbesondere über den OP-Monitor in Form eines Touchdisplays, und hat dann die Möglichkeit hiernach einen zweiten Punkt zu definieren, wobei diesen beiden gesetzten Punkte 14, 36 dann eine Gerade bzw. Strecke mit Nullpunkt 14 definieren, oder aber es steht auch die Option zur Verfügung, dass der Nutzer nach Definition des Nullpunkts 14 sein nachverfolgtes Instrument 10 dynamisch bewegt und zeitaktuell und dynamisch stets ein virtuelles Lineal 28 zwischen dem Nullpunkt 14 und der Instrumentenspitze 12 ausgerichtet und angezeigt wird.

Fig. 6 zeigt ein Flussdiagramm eines computerimplementierten chirurgischen intraoperativen Messverfahrens zur zeitaktuellen intraoperativen räumlichen Messung und Darstellung von geometrischen Streckenlängen oder Maßen oder Abständen einer bevorzugten Ausführungsform der vorliegenden Offenbarung.

In einem ersten optionalen Schritt S0 kann vorzugsweise ein Kalibrieren einer Visualisierungseinheit 2 erfolgen, die dafür angepasst ist eine intrakorporale Aufnahme 4 eines Eingriffsbereichs in einer Aufnahmeebene 5 zu erstellen.

In einem Schritt S1 erfolgt ein Erfassen S1 durch ein Navigationssystem 6, das dafür angepasst ist, über ein Trackingsystem 8 zumindest eine Instrumentenspitze 12 eines medizinischen Instruments (10) nachzuverfolgen, von zumindest einer Position der Instrumentenspitze 12 des navigierten Instruments 10.

In Schritt S2 erfolgt ein Erstellen durch eine Steuereinheit 20 einer digitalen Überlagerungsdarstellung 22 mit zumindest der intrakorporalen Aufnahme 4 sowie zumindest einer virtuellen Referenzdarstellung 24 der Instrumentenspitze 12 an der nachverfolgten Position, insbesondere einem Punkt oder Kreis als Referenzdarstellung der Instrumentenspitze 12, mit ferner einer virtuelle Messvorrichtung 26, insbesondere einem virtuellen Lineal 28, mit zumindest einer Abstandsmarkierung 32 und/oder einer Abstandszahl 34, die einem realen Abstand in der intrakorporalen Aufnahme 4 entspricht und dessen Nullpunkt die Instrumentenspitze bildet/ ist, wobei die virtuelle Messvorrichtung 26 parallel zu der Aufnahmeebene 5 angeordnet ist, insbesondere in der Aufnahmeebene 5 selbst liegt, um eine Abstandsmessung in der Aufnahmeebene 5 anzuzeigen, oder mittels dem nachverfolgten Instrument 10 neben dem Nullpunkt 14 noch ein räumlicher Endpunkt 36 definiert wird, welche beide die Gerade für die virtuelle Messvorrichtung 26 in Form eines virtuellen Lineals 28 bilden.

In Schritt S3 erfolgt das Ausgeben dieser erstellten Überlagerungsdarstellung 22 mit zumindest der virtuellen Referenzdarstellung 24 der Instrumentenspitze 12 und der virtuellen Messvorrichtung 26 über eine visuelle Darstellungsvorrichtung 16.

### Bezugszeichenliste

- 1: Chirurgisches intraoperatives Messsystem
- 2: Visualisierungseinheit
- 4: intrakorporale Aufnahme
- 5: Aufnahmeebene
- 6: Navigationssystem
- 8: Trackingsystem
- 10: Instrument
- 12: Instrumentenspitze
- 14: Nullpunkt
- 16: Darstellungsvorrichtung
- 18: OP-Monitor
- 20: Steuereinheit
- 22: Überlagerungsdarstellung
- 24: virtuelle Referenzdarstellung Instrumentenspitze
- 26: Virtuelle Messvorrichtung/ virtuelles Messinstrument
- 28: Virtuelles Lineal
- 30: Virtuelle Kreise
- 32: Abstandsmarkierung
- 34: Abstandszahl
- 36: Endpunkt
- 38: optisches Muster
- 40: Speichereinheit
- 42: Operationsmikroskop
- 44: Roboter
- 46: Roboterarm
- 48: Medizinischer Wagen
- 50: Navigationsansicht
- 52: Externe Kamera
- 54: zweites virtuelles koaxiales Lineal

- P: Patient
- 50: Schritt Kalibrierung Visualisierungsvorrichtung
- S1: Schritt Erfassen Position Instrumentenspitze
- S2: Schritt Erstellen Überlagerungsdarstellung mit virtueller Messvorrichtung
- S3: Schritt Ausgeben Überlagerungsdarstellung

## Patentansprüche

1. Chirurgisches intraoperatives Messsystem (1) zur zeitaktuellen intraoperativen räumlichen Messung und Darstellung von geometrischen Streckenlängen oder Maßen oder Abständen bei einer chirurgischen Navigation mit:
einer chirurgischen Visualisierungseinheit (2), die dafür angepasst ist eine intrakorporale Aufnahme (4) eines Eingriffsbereichs eines Patienten (P) in einer Aufnahmeebene (5) zu erstellen,
einem Navigationssystem (6), das dafür angepasst ist, über ein Trackingsystem (8) zumindest eine Instrumentenspitze (12) eines medizinischen Instruments (10) nachzuverfolgen, welches für den Eingriff im Eingriffsbereich einsetzbar ist, um mittels der Instrumentenspitze (12) einen Nullpunkt (14) für die Messung zu definieren, und
einer visuellen Darstellungsvorrichtung (16), insbesondere einem OP-Monitor (18), zur visuellen Ausgabe einer Darstellung mit zumindest der erstellten intrakorporalen Aufnahme (4),
**gekennzeichnet durch** eine Steuereinheit (20), die dafür angepasst ist:
- vorzugsweise die Visualisierungseinheit (2) zu kalibrieren,
- über das Navigationssystem (6) zumindest eine Position einer Instrumentenspitze (12) des navigierten Instruments (10) zu erfassen, insbesondere eine Lage des Instruments (10) mit Instrumentenspitze (12) zu erfassen,
- eine digitale Überlagerungsdarstellung (22) zu erstellen mit zumindest der intrakorporalen Aufnahme (4) sowie insbesondere einer virtuellen Referenzdarstellung (24) der Instrumentenspitze (12) an der nachverfolgten Position, insbesondere einem Punkt oder Kreis als Referenzdarstellung der Instrumentenspitze (24) in der intrakorporalen Aufnahme (4),
- in der erstellten Überlagerungsdarstellung (22) ferner eine virtuelle Messvorrichtung (26), insbesondere ein virtuelles Lineal (28), mit zumindest einer Abstandsmarkierung (32) und/oder einer Abstandszahl (34) einzufügen, die einem realen Abstand in der intrakorporalen Aufnahme (4) entspricht und dessen Nullpunkt (14) die Instrumentenspitze (12) bildet, wobei die virtuelle Messvorrichtung (26):
parallel zu der Aufnahmeebene (5) angeordnet ist, insbesondere in der Aufnahmeebene (5) selbst liegt, um eine Abstandsmessung in der Aufnahmeebene (5) anzuzeigen,
oder mittels dem nachverfolgten Instrument (10) neben dem Nullpunkt (14) noch ein räumlicher Endpunkt (36) definierbar ist, und die Verbindung beider Punkte (14, 36) eine Gerade für die virtuelle Messvorrichtung (26) in Form eines virtuellen Lineals (28) bilden, und
- diese erstellte Überlagerungsdarstellung (22) mit zumindest der virtuellen Messvorrichtung (26) über die Darstellungsvorrichtung (16) auszugeben.

2. Chirurgisches intraoperatives Messsystem (1) nach Anspruch 1, **dadurch gekennzeichnet, dass** das nachverfolgte Instrument (10) entweder im Bereich seiner Instrumentenspitze (12) ein vordefiniertes optisches Muster (38) und/oder eine vordefinierte geometrische Form aufweist, die in einer Speichereinheit (40) zum Abgleich hinterlegt ist, und die Steuereinheit (20) dafür angepasst ist auf Basis des erfassten optischen Musters (38) oder der geometrischen Form über die intrakorporale Aufnahme (4) der Visualisierungseinheit (2) und mittels Machine Vision die Position der Instrumentenspitze (12), insbesondere die Lage des Instruments (10), nachzuverfolgen.

3. Chirurgisches intraoperatives Messsystem (1) nach Anspruch 1 oder 2, **dadurch gekennzeichnet, dass** chirurgische Messsystem (1) als nachverfolgtes Instrument (10) einen Navigations-Zeiger und/oder einen Absaugschlauch und/oder ein chirurgisches Instrument, insbesondere ein Skalpell, aufweist.

4. Chirurgisches intraoperatives Messsystem (1) nach einem der vorstehenden Ansprüche, **dadurch gekennzeichnet, dass** das Messsystem (1) als Visualisierungseinheit (2) ein Operationsmikroskop (42) und/oder ein chirurgisches Exoskop und/oder ein chirurgisches Endoskop aufweist.

5. Chirurgisches intraoperatives Messsystem (1) nach einem der vorstehenden Ansprüche, **dadurch gekennzeichnet, dass** das Messsystem (1) ferner einen chirurgischen Roboter (44) aufweist, an dessen Roboterarm (46) die Visualisierungseinheit (2) navigiert und über die Steuereinheit (20) steuerbar angebunden ist.

6. Chirurgisches intraoperatives Messsystem (1) nach einem der vorstehenden Ansprüche, **dadurch gekennzeichnet, dass** das gesamte Messsystem in oder auf nur einem einzigen fahrbaren Wagen (48) integriert ist, um für eine gute Handhabung auf dem einzigen fahrbaren Wagen (48) ein vollständiges Messsystem (1) bereitzustellen.

7. Chirurgisches intraoperatives Messsystem (1) nach einem der vorstehenden Ansprüche, **dadurch gekennzeichnet, dass** in der Überlagerungsdarstellung(22) eine Nebeneinanderdarstellung von der intrakorporalen Aufnahme (4) und zudem noch einer Navigationsansicht (50) erstellt ist, wobei die virtuelle Messvorrichtung (26) in der intrakorporalen Aufnahme (4) und zudem auch noch lagekorrekt in der Navigationsansicht (50) eingeblendet ist, um für einen Nutzer zwei Ansichtsmodalitäten mit jedoch der gleichen virtuellen Messvorrichtung (26) bereitzustellen.

8. Chirurgisches intraoperatives Messsystem (1) nach einem der vorstehenden Ansprüche, **dadurch gekennzeichnet, dass** das Navigationssystem (6) eine externe Navigationskamera (52), insbesondere eine Infrarot-Navigationskamera für Infrarot-Tracker, aufweist.

9. Chirurgisches intraoperatives Messsystem (1) nach einem der vorstehenden Ansprüche, **dadurch gekennzeichnet, dass** das Messsystem (1) ferner eine Eingabeeinheit aufweist, insbesondere die Darstellungsvorrichtung (16) in Form eines Touchdisplays ausgebildet ist, und die Steuereinheit (20) dafür angepasst ist über eine Nutzereingabe der Eingabeeinheit:
- eine Abstandsbemaßung der virtuellen Messvorrichtung (26) einzustellen, insbesondere eine 1 mm oder 5mm Abstandsbemaßung einzustellen, und/oder
- eine geometrische Struktur der virtuellen Messvorrichtung (26) einzustellen, insbesondere in Form einer Lineallinie (28) mit Abstandsbemaßungen (32, 34) oder in Form von konzentrischen Kreisen (30), deren Radius sich in gleichen Abständen erhöht, und in der Aufnahmeebene (5) liegt.

10. Chirurgisches intraoperatives Messsystem (1) nach einem der vorstehenden Ansprüche, **dadurch gekennzeichnet, dass** die Steuereinheit (22) dafür angepasst ist, einen numerischen Abstand zwischen dem Nullpunkt (14) und dem Endpunkt (36) zu berechnen und diesen numerischen Abstand als Abstandszahl (34) mit einfügt.

11. Chirurgisches intraoperatives Messsystem (1) nach einem der vorstehenden Ansprüche, **dadurch gekennzeichnet, dass** die Steuereinheit (20) ferner dafür angepasst ist in der Überlagerungsdarstellung (22) neben der virtuellen Messvorrichtung (26) noch ein zweites virtuelles Lineal (54) in Verlängerung einer Längsachse des nachverfolgten Instruments (10) einzufügen.

12. Computerimplementiertes chirurgisches intraoperatives Messverfahren zur zeitaktuellen intraoperativen räumlichen Messung und Darstellung von geometrischen Streckenlängen oder Maßen oder Abständen, insbesondere für ein chirurgisches Messsystem (1) gemäß einem der Ansprüche 1 bis 11, **gekennzeichnet durch** die Schritte:
Vorzugsweise Kalibrieren (S0) einer Visualisierungseinheit (2), die dafür angepasst ist eine intrakorporale Aufnahme (4) eines Eingriffsbereichs in einer Aufnahmeebene (5) zu erstellen;
Erfassen (S1) durch ein Navigationssystem (6), das dafür angepasst ist, über ein Trackingsystem (8) zumindest eine Instrumentenspitze (12) eines medizinischen Instruments (10) nachzuverfolgen, von zumindest einer Position der Instrumentenspitze (12) des navigierten Instruments (10);
Erstellen (S2), durch eine Steuereinheit (20), einer digitalen Überlagerungsdarstellung (22) mit zumindest der intrakorporalen Aufnahme (4) sowie insbesondere einer virtuellen Referenzdarstellung (24) der Instrumentenspitze (12) an der nachverfolgten Position, insbesondere einem Punkt oder Kreis als Referenzdarstellung (24) der Instrumentenspitze (12), mit ferner einer virtuellen Messvorrichtung (26), insbesondere einem virtuellen Lineal (28), mit zumindest einer Abstandsmarkierung (32) und/oder einer Abstandszahl (34), die einem realen Abstand in der intrakorporalen Aufnahme (4) entspricht und dessen Nullpunkt die Instrumentenspitze (12) bildet, wobei die virtuelle Messvorrichtung (26) parallel zu der Aufnahmeebene (5) angeordnet ist, insbesondere in der Aufnahmeebene (5) selbst liegt, um eine Abstandsmessung in der Aufnahmeebene (5) anzuzeigen, oder mittels dem nachverfolgten Instrument (10) neben dem Nullpunkt (14) noch ein räumlicher Endpunkt (36) definiert wird, welche beide eine Gerade für die virtuelle Messvorrichtung (26) in Form eines virtuellen Lineals (28) bilden; und
Ausgeben (S3) dieser erstellten Überlagerungsdarstellung (22) mit zumindest der virtuellen Messvorrichtung (26) über eine visuelle Darstellungsvorrichtung (16).

13. Computerlesbares Speichermedium, das Befehle umfasst, die bei einer Ausführung durch einen Computer diesen veranlassen die Schritte des Messverfahrens nach Anspruch 12 auszuführen.

14. Computerprogramm, das Befehle umfasst, die bei einer Ausführung durch einen Computer diesen veranlassen die Schritte des Messverfahrens nach Anspruch 12 auszuführen.
